# EUROPEAN PATENT SPECIFICATION

(11) **EP 2 297 339 B1**
(45) Date of publication and mention of the grant of the patent: **12.04.2017**
(21) Application number: 09757400.8
(22) Date of filing: 20.05.2009
(51) Int. Cl.: C12Q 1/68

(54) **NUCLEIC ACIDS AND METHODS FOR DETECTING TURFGRASS PATHOGENIC FUNGI**
NUKLEINSÄUREN UND VERFAHREN ZUR DETEKTION VON PATHOGENEN PILZEN IN RASEN
Acides nucléiques et procédés de détection de champignons pathogènes de pelouse en plaque

(30) Priority: 02.06.2008 EP 08104225; 09.06.2008 US 59862 P
(43) Date of publication of application: 23.03.2011
(60) Divisional application: 15150835.5
(73) Proprietor: Omya International AG, 4665 Oftringen (CH)
(72) Inventor: DI MAIUTA, Nicola, CH-4528 Zuchwil (CH); SCHWARZENTRUBER, Patrick, CH-5245 Habsburg (CH)
(74) Representative: Lavoix
(86) International application number: PCT/EP2009/056189
(87) International publication number: WO 2009/147017

(56) References cited:
- EP-A- 0 859 061
- WO-A-2004/040017
- JP-A- 2008 005 760
- DATABASE GENBANK 2003, GATHMAN: "Expressed sequence tags from Schizophyllum commune nitrogen-replete and nitrogen-limited libraries" XP002500783 retrieved from NCBI Database accession no. cf800463
- DATABASE GENBANK 1992, MITCHELL, WHITE, TAYLOR: "Filobasidium uniguttulatum 5.8S ribosomal RNA gene and internal transcribed spacer 2, partial sequence" XP002500784 retrieved from NCBI Database accession no. M94520

## Description

### Related applications

The present application claims the benefit of EP patent application serial No. 08104225.1 and US provisional application serial No. 61/059862.

### Field of the invention

The present invention relates to nucleic acids and methods for detecting organic substrate pathogenic fungi.

### Background of the invention

Disease in lawngrasses or turfgrasses develops from an interaction among a susceptible plant, an environment favorable for disease development, and a pathogenic organism, usually a fungus. Such fungi may also develop on decorative grasses, plants and crops; indeed, they may appear on any suitable organic substrate. Thus, treatment of a diseased substrate, especially turfgrass, usually consists in applying fungicides that will either kill the fungus or keep it from growing.

However, the first step in disease management, and especially turfgrass disease management, should always consist in identifying the causative pathogenic agent. Indeed, it is important to have identified the disease correctly, so that an appropriate fungicide can be selected. Arbitary selection and application of fungicides without knowledge of the disease cause can do as much harm as good. Using the wrong fungicide wastes money and may involve the risk of exacerbating the disease, as well as causing other unwanted side effects.

Classical methods for the identification of the causative pathogenic agent essentially rely on the symptoms which can be observed on the individual plant and on the turf stand, as well as on the fungal structures, such as mycelia or spores, which can be found in the vicinity of the diseased turfgrass.

However, these methods may require a long time to be implemented, since they often involve the isolation and the culture of the fungi in a laboratory. Besides, differentiating closely related fungal species can prove difficult.

Accordingly, molecular biology methods have been developped which circumvent these difficulties. One of the most popular fungal detection methods relies on the PCR amplification of the internal transcribed spacers (1, 2) and the 5.8S rRNA gene (ITS1-5.8S-ITS2) from the fungal rRNA operon (Goodwin et al. (1995) Plant Pathology 44:384-391; Ranjard et al. (2001) Applied and Environmental Microbiology 67:4479-4487). However, a specific primer pair is often necessary for the indentification of a given fungal species, which renders this method cumbersome where the identity of pathogenic fungus is unknown and is sought for. Furthermore, some fungal species can not be differentiated using the primers currently available which target this region. Accordingly, this method is not used in routine for determining the antifungal agent most adapted to treat a given turfgrass disease.

Japanese patent application No. 2008005760 discloses 458 probes for detecting molds that can be found in food. These 458 probes are designed for detecting molds by a hybridization-based method involving the use of a microarray.

It is therefore an object of the invention to provide a method which allows the rapid specific detection of nucleic acids from the fungi most commonly involved in turfgrass diseases.

### Summary of the invention

The present invention arises from the identification, by the inventors, of a conserved region within the rRNA operon of the genome of pathogenic fungi generally affecting turfgrasses, but also possibly other organic substrates, which is liable to be used as a target in the frame of nucleic acid amplification-based detection method of most of such pathogenic fungi.

Thus the present invention relates to the use of at least one nucleic acid comprising or consisting of:
(i) wherein:
   R represents A or G
   Y represents C or T
   M represents A or C
   W represents A or T
   H represents A or C or T, or
(ii) complementary sequences of (i);
   for the detection of nucleic acids from one or more fungi in a sample.

In a preferred embodiment, the present invention more particularly relates to the use of at least one nucleic acid comprising or consisting of:
(i) GTGARTCATCGAAWYTTTGAACGCA (SEQ ID NO: 2), wherein:
   R represents A or G
   Y represents C or T
   W represents A or T
(ii) a portion of SEQ ID NO: 2, provided said nucleic acid binds under stringent conditions to a nucleic acid comprising or consisting of the complementary sequence of SEQ ID NO: 1, or
(iii) complementary sequences of (i) and (ii);
for the detection of nucleic acids from one or more fungi in a sample.

The present invention also relates to a method for detecting nucleic acids from one or more fungi in a sample, wherein at least one nucleic acid comprising or consisting of:
(i) wherein:
   R represents A or G
   Y represents C or T
   M represents A or C
   W represents A or T
   H represents A or C or T
(ii) a portion of SEQ ID NO: 1, provided said nucleic acid binds under stringent conditions to a nucleic acid comprising or consisting of the complementary sequence of SEQ ID NO: 1, or
(iii) complementary sequences of (i) and (ii); is contacted with the sample.

In a preferred embodiment, the invention more particularly relates to a method for detecting nucleic acids from one or more fungi in a sample, wherein at least one nucleic acid comprising or consisting of:
(i) GTGARTCATCGAAWYTTTGAACGCA (SEQ ID NO: 2), wherein:
   R represents A or G
   Y represents C or T
   W represents A or T
(ii) a portion of SEQ ID NO: 2, provided said nucleic acid binds under stringent conditions to a nucleic acid comprising or consisting of the complementary sequence of SEQ ID NO: 1, or
(iii) complementary sequences of (i) and (ii);
is contacted with the sample.

The present invention also relates to a method for treating a diseased organic substrate, which comprises the steps of:
a) detecting the absence or the presence of nucleic acids from at least one pathogenic fungus in a sample of the substrate, with at least one nucleic acid according to the invention as defined above;
b) if nucleic acids from one or more pathogenic fungi have been detected in step a), selecting one or more antifungal agents which target the one or more pathogenic fungi from which nucleic acids have been detected;
c) applying the selected one or more antifungal agents of step b) to the diseased substrate.

The present invention also relates to a kit for the detection of fungi, comprising each one of the nucleic acids represented by:
(i) wherein:
   R represents A or G
   Y represents C or T
   M represents A or C
   W represents A or T
   H represents A or C or T
(ii) a portion of SEQ ID NO: 1, provided said nucleic acid binds under stringent conditions to a nucleic acid comprising or consisting of the complementary sequence of SEQ ID NO: 1, or
(iii) complementary sequences of (i) and (ii).

In a preferred embodiment, the invention more particularly relates to a kit for the detection of fungi, comprising each one of the nucleic acids represented by:
(i) GTGARTCATCGAAWYTTTGAACGCA (SEQ ID NO: 2), wherein:
   R represents A or G
   Y represents C or T
   W represents A or T
(ii) a portion of SEQ ID NO: 2, provided said nucleic acid binds under stringent conditions to a nucleic acid comprising or consisting of the complementary sequence of SEQ ID NO: 1, or
(iii) complementary sequences of (i) and (ii).

The present invention also relates to a nucleic acid comprising or consisting of:
(i) wherein:
   R represents A or G
   Y represents C or T
   M represents A or C
   W represents A or T
   H represents A or C or T
(ii) a portion of SEQ ID NO: 1, provided the said nucleic acid binds under stringent conditions to a nucleic acid comprising or consisting of the complementary sequence of SEQ ID NO: 1, or
(iii) complementary sequences of (i) and (ii).

In a preferred embodiment, the invention more particularly relates to a nucleic acid comprising or consisting of:
(i) GTGARTCATCGAAWYTTTGAACGCA (SEQ ID NO: 2), wherein:
   R represents A or G
   Y represents C or T
   W represents A or T
(ii) a portion of SEQ ID NO: 2, provided said nucleic acid binds under stringent conditions to a nucleic acid comprising or consisting of the complementary sequence of SEQ ID NO: 1, or
   complementary sequences of (i) and (ii).

In another preferred embodiment, the invention more particularly relates to a nucleic acid as defined above, comprising or consisting of a sequence selected from the group consisting of SEQ ID NO: 1 to 38, or complementary sequences thereof.

### Detailed description of the invention

Nucleic acids as intended herein can be of any type, however it is preferred that they be DNA.

"Stringent conditions" can be easily be defined by the man skilled in the art using common knowledge. If necessary, guidance for defining such conditions can be found in numerous textbooks, such as Tijssen, 1993, Laboratory Techniques in Biochemistry and Molecular Biology - Hybridization with Nucleic Acid Probes, Part I, Chapter 2 "Overview of principles of hybridization and the strategy of nucleic acid probe assays", Elsevier, New York. Preferably, stringent conditions according to the invention are constituted of an annealing temperature of 60°C carried out in a PCR reaction medium comprising, *e.g.* 50 mM KCl, 1.5 mM MgCl₂ and 10 mM Tris pH 8.3.

As intended herein a "portion" of nucleic acid preferably comprises a number of nucleotides sufficient to provide for a specific hybridisation to the nucleic acid comprising or consisting of the complementary sequence of SEQ ID NO: 1. Thus, the portion of nucleic acid preferably comprises at least 9 nucleotides, more preferably at least 15 nucleotides, even more preferably at least 20 nucleotides, and most preferably at least 25 nucleotides.

As intended herein, it is preferred that the maximum length of the nucleic acids according to the invention is less than 500 nucleotides.

Preferably, the at least one nucleic acid as defined above is used as a primer in a nucleic acid amplification-based detection method, such as Amplification Fragment Length Polymorphism (AFLP) or Terminal Restriction Fragment Length Polymorphism (TRFLP or T-RFLP).

Nucleic acid amplification-based detection methods are particularly well known to one of skill in the art. Terminal Restriction Fragment Length Polymorphism (TRFLP or T-RFLP), for instance, is notably described by Formey et al. (1997) Applied and Environmental Microbiology 63:4516-4522.

Where the at least one nucleic acid as defined above is used as a primer, it is preferably used in association with at least one other primer. The other primer can be any primer targeting a sequence comprised within the genome of the one or more fungi. However, it is preferred that the other primer targets the 18S rDNA/ITS1 region or the ITS2/28S rDNA region of the one or more fungi.

The fungal rRNA operon comprises the 18S rRNA gene, the Internal Transcribed Spacer 1 (ITS1), the 5.8S rRNA gene, the Internal Transcribed Spacer 2 (ITS2), and the 28S rRNA gene. The 18S rDNA/ITS1 and ITS2/28S rDNA regions thus relate to the regions of the genome of the one or more fungi in the vicinity respectively of the junction of the 18S rRNA gene and ITS1, and of the junction of TIS2 and the 28S rRNA gene. As will be clear to anyone of skill in the art, where the at least one nucleic acid as defined above is used as a forward primer, the primer which targets the 18S rDNA/ITS1 region or the ITS2/28S rDNA region will be a reverse primer, and *vice versa.* The sequences of the 18S rDNA/ITS1 and ITS2/28S rDNA regions are well known to one of skill in the art and can usually be accessed from public sequence databases. Where sequences of the 18S rDNA/ITS1 and ITS2/28S rDNA regions would not be publicly available for a particular fungus species, they can be routinely sequenced. Besides, it is well within the common knowledge of anyone of skill in the art to select primers within the known sequences.

By way of example of a primer targeting the 18S rDNA/ITS1 region, one can cite the so-called "ITS1-F primer" of sequence TCCGTAGGTGAACCTGCGG (SEQ ID NO: 39). Conversely, by way of example of a primer targeting the ITS2/28S rDNA region one can cite the so-called "ITS4 primer" of sequence TCCTCCGCTTATTGATATGC (SEQ ID NO: 40). Other examples of primers liable to be used with the primers according to the invention include the "ITS5 primer" of sequence GGAAGTAAAAGTCGTAACAAGG (SEQ ID NO: 41) and the "SR6R primer" of sequence AAGWAAAAGTCGTAACAAGG (SEQ ID NO: 42). Still other Examples are available from http://www.biology.duke.edu/fungi/mycolab/primers.htm.

As intended herein the primers to be used may be unmodified or modified nucleic acids, in particular DNA. Where the primers are modified nucleic acids they can notably be labelled nucleic acids, in particular fluorescently labelled nucleic acids.

Where TRFLP is used, the one of skill in the art knows how to design primers and to select restriction enzymes so that the nucleic acid generated and fragmented by the enzyme presents a fragment length that can be detected. In this regard, it should be noted that detection of a fragment is dependant on the instrumentation and detection techniques used, but in general detection is possible for sequences featuring at least 15 nucleotides. Besides, the length of the fragment should preferably be such that it is distinguishable from fragment lengths generated by the same primer pair/enzyme in other fungi (in general, fragment lengths differing by more than 1, and preferably more than 2, nucleotides are distinguished by current techniques). Numerous databases and tools are available to one of skill in the art for selecting primers and restriction enzymes, such as the REBASE database.

In the case where nucleic acids according to the invention include nucleotides featuring a "wobble" position, that is to say they include nucleotides that may be selected from among two or more possible nucleotides, one of skill in the art knows that it is usually advantageous to use a mixture of primers such that all of the nucleotide possibilities are represented.

Thus, by way of example, each one of the nucleic acids represented by GTGARTCATCGAAWYTTTGAACGCA (SEQ ID NO: 2), are as follows:
GTGAATCATCGAAACTTTGAACGCA (SEQ ID NO: 3);
GTGAGTCATCGAAACTTTGAACGCA (SEQ ID NO: 4);
GTGAATCATCGAATCTTTGAACGCA (SEQ ID NO: 5);
GTGAGTCATCGAATCTTTGAACGCA (SEQ ID NO: 6);
GTGAATCATCGAAATTTTGAACGCA (SEQ ID NO: 7);
GTGAGTCATCGAAATTTTGAACGCA (SEQ ID NO: 8);
GTGAATCATCGAATTTTTGAACGCA (SEQ ID NO: 9);
GTGAGTCATCGAATTTTTGAACGCA (SEQ ID NO: 10);
GTGAATCATCGAAACTTTGAACGCA (SEQ ID NO: 11);
GTGAGTCATCGAAACTTTGAACGCA (SEQ ID NO: 12);
GTGAATCATCGAATCTTTGAACGCA (SEQ ID NO: 13);
GTGAGTCATCGAATCTTTGAACGCA (SEQ ID NO: 14);
GTGAATCATCGAAATTTTGAACGCA (SEQ ID NO: 15);
GTGAGTCATCGAAATTTTGAACGCA (SEQ ID NO: 16);
GTGAATCATCGAATTTTTGAACGCA (SEQ ID NO: 17);
GTGAGTCATCGAATTTTTGAACGCA (SEQ ID NO: 18).

As intended herein, it is to be understood that the invention aims at the detection of nucleic acids of fungi of any type, however it is preferred that the fungus is a plant pathogenic fungus, in particular a turfgrass pathogenic fungus, such as a fungus selected from the group constituted of *Ascochyta phleina, Curvularia affinis, Glomerella graminicola, Thanatephorus cucumeris, Pythium ultimum, Gaeumannomyces graminis, Marasmius oreades, Corticium fuciforme, Phytophthora nicotianae. Fusarium culmorum, Bipolaris sorokiniana, Microdochium nivale, Rhizoctonia cerealis, Pythium graminicola, Rhynchosporium secalis, Sclerotinia homoeocarpa, Typhula incarnate, Ustilago striiformis, Septoria macropoda.*

As intended herein the sample in which nucleic acids are to be detected can be of any type of organic substrate liable to contain nucleic acids from fungi. However, it is preferred that the sample be a turfgrass sample or a soil sample.

Where the sample is a turfgrass sample, it can be a sample obtained from the turfgrass as a whole or from or a sample of a part of the turfgrass, such as the root.

Where the sample is a soil sample, it is preferably taken directly under the diseased turfgrass or in the vicinity of the diseased turgrass.

The sample can be obtained directly from turfgrass or soil, or be obtained after treatment steps, such as grinding or extraction, in particular nucleic acid extraction, steps.

As intended herein, any diseased turfgrass can be subjected the use or methods as defined above. Preferred turfgrasses to be considered within the frame of the present invention are notably described in http://www.ars-grin.gov/cgi-bin/npgs/html/index.pl, from the Germplasm Resources Information Network, National Germplasm Resources Laboratory, Beltsville, Maryland, or in the Compendium of Turgrass Diseases, Third Edition (2005) by the American Phytopathological Society. Most preferably, the diseased turfgrass according to the invention is selected from the group consisting of the *Festaceae, Aveneae, Triticeae, Chlorideae, Zoysieae, Paniceae* and *Andropogoneae Tribe.*

In a specific embodiment, nucleic acids comprising or consisting of the sequences of SEQ ID Nos. 159, 188, 198, 221, 263 and/or 416 of Japanese patent application No. 2008005760 are excluded from the scope of the present invention.

### EXAMPLE

The following turfgrass pathogenic fungus species were sequenced by the inventors: *Ascochyta phleina, Curvularia affinis, Glomerella graminicola, Thanatephorus cucumeris, Pythium ultimum, Gaeumannomyces graminis, Marasmius oreades, Corticium fuciforme, Phytophthora nicotianae. Fusarium culmorum, Bipolaris sorokiniana, Microdochium nivale, Rhizoctonia cerealis, Pythium graminicola, Rhynchosporium secalis, Sclerotinia homoeocarpa, Typhula incarnate, Ustilago striiformis, Septoria macropoda.*

Briefly, the well-known sequencing method developed by Fred Sanger - chain termination method - was used using Applied Biosystem BigDye® Terminator Cycle Sequencing v1 or v3.1 chemistry on an Applied Biosystems Genetic Analyzer 3130. The sequences were gathered by using the ITS1 forward primer (ITS1-F, TCCGTAGGTGAACCTGCGG, SEQ ID NO: 39).

The obtained sequences are represented by SEQ ID NO: 19 to 37.

From these sequences a particular consensus sequence could be determined by the inventors:

The discriminative potential of this region was then evidenced by *in silico* Terminal Restriction Fragment Length Polymorphism (TRFLP or T-RFLP) by using the primer ITSOMYAr (TGCGTTCAAARWTTCGATGAYTCAC, SEQ ID NO: 38, the complementary of SEQ ID NO: 2), which hybridizes to the above consensus sequence, in association with the above ITS1-F primer.

The applied enzymes for the T-RFLP analysis were *Taq*l (T^{▼}CG_{▲}A) and *Tsp*509l (^{▼}AATT_{▲}).

The following tables show the PCR fragment length of both primer combination ITS1-F/ITS4 (full length ITS) (ITS4, TCCTCCGCTTATTGATATGC, SEQ ID NO: 40) and ITS1 /ITSOMYAr (partial ITS length) (Table 1), as well as the corresponding *in silico* and *in vitro* digested partial ITS (ITS1/ITSOMYAr) fragments by *Taq*l (Table 2) and *Tsp*509l (Table 3) (mean and standard deviation (StDev) were calculated from triplicate measurements of two independent PCR assays).

**Table 1**

| | **Fragments [bps]** | |
|---|---|---|
| **Fungal name** | **Full ITS length ITS1/ITS4** | **Partial ITS length ITS1/ITSOMYAr** |
| *Ascochyta phleina* | 588 | 326 |
| *Curvularia affinis* | 584 | 316 |
| *Glomerella graminicola* | 587 | *320* |
| *Thanatephorus cucumeris* | 710 | *360* |
| *Pythium ultimum* | 914 | *360* |
| *Gaeumannomyces graminis* | 558 | *280* |
| *Marasmius oreades* | 672 | 355 |
| *Corticium fuciforme* | 716 | 353 |
| *Phytophthora nicotianae* | 892 | 367 |
| *Fusarium culmorum* | 545 | 286 |
| *Bipolaris sorokiniana* | 586 | 309 |
| *Microdochium nivale* | 556 | 283 |
| *Rhizoctonia cerealis* | 684 | 348 |
| *Pythium graminicola* | 872 | 319 |
| *Rhynchosporium secalis* | 627 | 371 |
| *Sclerotinia homoeocarpa* | 586 | *320* |
| *Typhula incarnata* | 825 | 410 |
| *Ustilago striiformis* | 780 | *380* |
| *Septoria macropoda* | 540 | 277 |

**Table 2**

| | ***Taql fragments [bps]*** | | |
|---|---|---|---|
| | ***in-silico*** | **measured** | |
| **Fungal name** | | **mean** | **StDev** |
| *Ascochyta phleina* | 252 | 247.0 | 0.022 |
| *Curvularia affinis* | 242 | 236.4 | 0.048 |
| *Glomerella graminicola* | 246 | 239.3 | 0.113 |
| *Thanatephorus cucumeris* | *286* | 284.4 | 0.123 |
| *Pythium ultimum* | *286* | 284.0 | 0.043 |
| *Gaeumannomyces graminis* | 206 | 198.9 | 0.030 |
| *Marasmius oreades* | 281 | 279.6 | 0.057 |
| *Corticium fuciforme* | 279 | 275.7 | 0.054 |
| *Phytophthora nicotianae* | 293 | 290.9 | 0.056 |
| *Fusarium culmorum* | 212 | 207.9 | 0.033 |
| *Bipolaris sorokiniana* | 235 | 230.2 | 0.054 |
| *Microdochium nivale* | 209 | 206.5 | 0.247 |
| *Rhizoctonia cerealis* | 71 | 67.7 | 0.049 |
| *Pythium graminicola* | 245 | 242.8 | 0.033 |
| *Rhynchosporium secalis* | 113 | 108.5 | 0.043 |
| *Sclerotinia homoeocarpa* | 102 | 98.1 | 0.043 |
| *Typhula incarnata* | 32 | 29.9 | 0.182 |
| *Ustilago striiformis* | 31 | 29.6 | 0.130 |
| *Septoria macropoda* | 162 | 147.2 | 0.161 |

**Table 3**

| | ***Tsp509*/ *fragments [bps]*** | | |
|---|---|---|---|
| | ***in-silico*** | **measured** | |
| **Fungal name** | | **mean** | **StDev** |
| *Ascochyta phleina* | 167 | 163.5 | 0.041 |
| *Curvularia affinis* | 278 | 272.1 | 0.044 |
| *Glomerella graminicola* | 207 | 201.2 | 0.034 |
| *Thanatephorus cucumeris* | 34 | 32.3 | 0.091 |
| *Pythium ultimum* | 322 | 321.4 | 0.040 |
| *Gaeumannomyces graminis* | 134 | 125.7 | 0.096 |
| *Marasmius oreades* | 225 | 223.9 | 0.056 |
| *Corticium fuciforme* | 315 | 312.8 | 0.042 |
| *Phytophthora nicotianae* | 108 | 104.1 | 0.026 |
| *Fusarium culmorum* | 248 | 243.3 | 0.029 |
| *Bipolaris sorokiniana* | 193 | 189.0 | 0.032 |
| *Microdochium nivale* | 137 | 133.5 | 0.015 |
| *Rhizoctonia cerealis* | 310 | 307.6 | 0.032 |
| *Pythium graminicola* | 64 | 58.6 | 0.048 |
| *Rhynchosporium secalis* | 102 | 329.7 | 0.030 |
| *Sclerotinia homoeocarpa* | 175 | 172.0 | 0.066 |
| *Typhula incarnata* | 34 | 31.9 | 0.112 |
| *Ustilago striiformis* | 143 | 140.7 | 0.013 |
| *Septoria macropoda* | 239 | 222.4 | 0.169 |

In general, since the length of the fragments yielded by the ITS1/ITSOMYAr combination, either in AFLP or in T-RFLP, is usually smaller than the corresponding fragment obtained using the ITS1/ITS4 combination, the fragments can be separated with a higher resolution.

Furthermore, it can be seen that the ITS1/ITSOMYAr combination provides, either in AFLP or in T-RFLP, a good alternative to the ITS1/ITS4 combination in AFLP since it notably enables to discriminate between species which discrimination was either impossible or very difficult to carry out with the ITS1/ITS4 combination (*e.g. Ascochyta phleina, Curvularia affinis, Glomerella graminicola, Bipolaris sorokiniana, Sclerotinia homoeocarpa).*

## Claims

1. The use of at least one nucleic acid comprising or consisting of:
(i) wherein:
R represents A or G
Y represents C or T
M represents A or C
W represents A or T
H represents A or C or T
(ii) a portion of SEQ ID NO: 1, provided said nucleic acid binds under stringent conditions to a nucleic acid comprising or consisting of the complementary sequence of SEQ ID NO: 1, or
(iii) complementary sequences of (i) and (ii);
as a primer for the detection by Amplification Fragment Length Polymorphism (AFLP) or Terminal Restriction Fragment Length Polymorphism (T-RFLP) of nucleic acids from one or more fungi in a turfgrass or a soil sample, the at least one nucleic acid being used in association with at least one other primer which targets the 18S rDNA/ITS1 region or the ITS2/28S rDNA region.

2. The use according to claim 1, of at least one nucleic acid comprising or consisting of:
(i) GTGARTCATCGAAWYTTTGAACGCA (SEQ ID NO: 2), wherein:
R represents A or G
Y represents C or T
W represents A or T
(ii) a portion of SEQ ID NO: 2, provided said nucleic acid binds under stringent conditions to a nucleic acid comprising or consisting of the complementary sequence of SEQ ID NO: 1, or
(iii) complementary sequences of (i) and (ii).

3. The use according to claim 1 or 2, wherein the fungus is selected from the group constituted of *Ascochyta phleina, Curvularia affinis, Glomerella graminicola, Thanatephorus cucumeris, Pythium ultimum, Gaeumannomyces graminis, Marasmius oreades, Corticium fuciforme, Phytophthora nicotianae. Fusarium culmorum, Bipolaris sorokiniana, Microdochium nivale, Rhizoctonia cerealis, Pythium graminicola, Rhynchosporium secalis, Sclerotinia homoeocarpa, Typhula incarnate, Ustilago striiformis, Septoria macropoda.*

4. The use according to any of claims 1 to 3, wherein the sample is a turfgrass root sample.

5. The use according to any of claims 1 to 4, wherein the turfgrass is selected from the group consisting of the *Festaceae, Aveneae, Triticeae, Chlorideae, Zoysieae, Paniceae and Andropogoneae* Tribe.

6. The use according to any one of the preceding claims, wherein the other primer is selected from the list constituted of SEQ ID NO: 39 to 42.

7. A method for treating a diseased turfgrass, which comprises the steps of:
a) detecting by Amplification Fragment Length Polymorphism (AFLP) or Terminal Restriction Fragment Length Polymorphism (T-RFLP) the absence or the presence of nucleic acids from at least one pathogenic fungus in a sample of soil in which the diseased turfgrass is growing, or in a sample of the diseased turfgrass, with at least one nucleic acid as defined in claim 1 or 2 as a primer, the at least one nucleic acid being used in association with at least one other primer which targets the 18S rDNA/ITS1 region or the ITS2/28S rDNA region;
b) If nucleic acids from one or more pathogenic fungi have been detected in step a), selecting one or more antifungal agents which target the one or more pathogenic fungi from which nucleic acids have been detected;
c) Applying the selected one or more antifungal agents of step b) to the diseased turfgrass.

8. The method according to claim 7, wherein the fungus is selected from the group constituted of *Ascochyta phleina, Curvularia affinis, Glomerella graminicola, Thanatephorus cucumeris, Pythium ultimum, Gaeumannomyces graminis, Marasmius oreades, Corticium fuciforme, Phytophthora nicotianae. Fusarium culmorum, Bipolaris sorokiniana, Microdochium nivale, Rhizoctonia cerealis, Pythium graminicola, Rhynchosporium secalis, Sclerotinia homoeocarpa, Typhula incarnate, Ustilago striiformis, Septoria macropoda.*

9. The method according to claim 7 or 8, wherein the diseased turfgrass is selected from the group consisting of the *Festaceae, Aveneae, Triticeae, Chlorideae, Zoysieae, Paniceae and Andropogoneae* Tribe.

10. The method according to any one of claims 7 to 9, wherein the other primer is selected from the list constituted of SEQ ID NO: 39 to 42.

11. A kit for the detection of fungi, comprising each one of the nucleic acids represented by:
(i) wherein:
R represents A or G
Y represents C or T
M represents A or C
W represents A or T
H represents A or C or T, or
(ii) complementary sequences of (i).

12. The kit according to claim 11, comprising the nucleic acids represented by the following sequences:
GTGAATCATCGAAACTTTGAACGCA (SEQ ID NO: 3);
GTGAGTCATCGAAACTTTGAACGCA (SEQ ID NO: 4);
GTGAATCATCGAATCTTTGAACGCA (SEQ ID NO: 5);
GTGAGTCATCGAATCTTTGAACGCA (SEQ ID NO: 6);
GTGAATCATCGAAATTTTGAACGCA (SEQ ID NO: 7);
GTGAGTCATCGAAATTTTGAACGCA (SEQ ID NO: 8);
GTGAATCATCGAATTTTTGAACGCA (SEQ ID NO: 9);
GTGAGTCATCGAATTTTTGAACGCA (SEQ ID NO: 10);
GTGAATCATCGAAACTTTGAACGCA (SEQ ID NO: 11);
GTGAGTCATCGAAACTTTGAACGCA (SEQ ID NO: 12);
GTGAATCATCGAATCTTTGAACGCA (SEQ ID NO: 13);
GTGAGTCATCGAATCTTTGAACGCA (SEQ ID NO: 14);
GTGAATCATCGAAATTTTGAACGCA (SEQ ID NO: 15);
GTGAGTCATCGAAATTTTGAACGCA (SEQ ID NO: 16);
GTGAATCATCGAATTTTTGAACGCA (SEQ ID NO: 17);
GTGAGTCATCGAATTTTTGAACGCA (SEQ ID NO: 18);
or their complementary sequences.

## Patentansprüche

1. Verwendung von wenigstens einer Nukleinsäure, umfassend oder bestehend aus:
(i) worin:
R A oder G bedeutet,
Y C oder T bedeutet,
M A oder C bedeutet,
W A oder T bedeutet,
H A oder C oder T bedeutet
(ii) einem Teil von SEQ ID Nr: 1, vorausgesetzt, dass die Nukleinsäure unter stringenten Bedingungen an eine Nukleinsäure bindet, welche die komplementäre Sequenz von SEQ ID Nr: 1 umfasst oder aus der komplementären Sequenz von SEQ ID Nr: 1 besteht, oder
(iii) komplementären Sequenzen von (i) und (ii);
als ein Primer für die Detektion von Nukleinsäuren aus einem oder mehreren Pilzen in einer Rasengras- oder Bodenprobe durch amplifizierten Fragmentlängenpolymorphismus (AFLP) oder durch terminalen Restriktionsfragmentlängenpolymorphismus (T-RFLP), wobei die wenigstens eine Nukleinsäure in Verbindung mit wenigstens einem anderen Primer, welches auf die 18S rDNA/ITS Region oder die ITS2/28S rDNA Region zielt, verwendet wird.

2. Verwendung nach Anspruch 1 von wenigstens einer Nukleinsäure, umfassend oder bestehend aus:
(i) GTGARTCATCGAAWYTTTGAACGCA (SEQ ID Nr: 2), worin
R A oder G bedeutet
Y C oder T bedeutet
W oder T bedeutet
(ii) einem Teil von SEQ ID Nr: 2, vorausgesetzt, dass die Nukleinsäure unter stringenten Bedingungen an eine Nukleinsäure bindet, welche die komplementäre Sequenz von SEQ ID Nr: 1 umfasst oder aus der komplementären Sequenz von SEQ ID Nr: 1 besteht, oder
(iii) komplementäre Sequenzen von (i) und (ii).

3. Verwendung nach Anspruch 1 oder 2, bei der der Pilz ausgewählt ist aus der Gruppe gebildet aus *Ascochyta phleina, Curvularia affinis, Glomerella graminicola, Thanatephorus cucumeris, Pythium ultimum, Gaeumannomyces graminis, Marasmius oreades, Corticium fuciforme, Phytophthora nicotianae. Fusarium culmorum, Bipolaris sorokiniana, Microdochium nivale, Rhizoctonia cerealis, Pythium graminicola, Rhynchosporium secalis, Sclerotinia homoeocarpa, Typhula incarnate, Ustilago strüformis, Septoria macropoda.*

4. Verwendung nach einem der Ansprüche 1 bis 3, bei der die Probe eine Rasengraswurzelprobe ist.

5. Verwendung nach einem der Ansprüche 1 bis 4, bei der das Rasengras ausgewählt ist aus der Gruppe bestehend aus dem *Festaceae, Aveneae, Triticeae, Chlorideae, Zoysieae, Paniceae and Andropogoneae* Tribus.

6. Verwendung nach einem der vorhergehenden Ansprüche, bei der der andere Primer ausgewählt ist aus der Liste gebildet aus SEQ ID Nr: 39 bis 42.

7. Verfahren zur Behandlung von erkranktem Rasengras, das die Schritte umfasst:
a) Detektieren der Abwesenheit oder der Gegenwart von Nukleinsäuren aus wenigstens einem pathogenen Pilz in einer Bodenprobe, in der das erkrankte Rasengras wächst, oder in einer Probe des erkrankten Rasengrases durch amplifizierten Fragmentlängenpolymorphismus (AFLP) oder durch terminalen Restriktionsfragmentlängenpolymorphismus (T-RFLP), mit wenigstens einer Nukleinsäure wie in Anspruch 1 oder 2 definiert als Primer, wobei die wenigstens eine Nukleinsäure in Verbindung mit wenigstens einem anderen Primer, welches auf die 18S rDNA/ITS1 Region oder die ITS2/28S rDNA Region zielt, verwendet wird;
b) wenn Nukleinsäuren aus einem oder mehreren pathogenen Pilzen in Schritt a) detektiert wurden, Auswählen von einem oder mehreren Pilzmitteln, welche auf die einen oder mehreren pathogenen Pilze zielen, aus denen die Nukleinsäuren detektiert wurden;
c) Anwenden der einen oder mehreren ausgewählten Pilzmittel aus Schritt b) auf das erkrankte Rasengras.

8. Verfahren nach Anspruch 7, bei dem der Pilz ausgewählt ist aus der Gruppe gebildet aus *Ascochyta phleina, Curvularia affinis, Glomerella graminicola, Thanatephorus cucumeris, Pythium ultimum, Gaeumannomyces graminis, Marasmius oreades, Corticium fuciforme, Phytophthora nicotianae. Fusarium culmorum, Bipolaris sorokiniana, Microdochium nivale, Rhizoctonia cerealis, Pythium graminicola, Rhynchosporium secalis, Sclerotinia homoeocarpa, Typhula incarnate, Ustilago striiformis, Septoria macropoda.*

9. Verfahren nach Anspruch 7 oder 8, bei dem das erkrankte Rasengras ausgewählt ist aus der Gruppe bestehend aus dem *Festaceae, Aveneae, Triticeae, Chlorideae, Zoysieae, Paniceae and Andropogoneae* Tribus.

10. Verfahren nach Anspruch 7 bis 9, bei dem der andere Primer ausgewählt ist aus der Liste gebildet aus SEQ Nr: 39 bis 42.

11. Kit für die Detektion von Pilzen, umfassend jede der Nukleinsäuren dargestellt durch:
(i) worin:
R A oder G bedeutet,
Y C oder T bedeutet,
M A oder C bedeutet,
W A oder T bedeutet,
H A oder C oder T bedeutet, oder
(ii) komplementäre Sequenzen von (i).

12. Kit nach Anspruch 11, umfassend die Nukleinsäuren, die durch die folgenden Sequenzen dargestellt sind:
GTGAATCATCGAAACTTTGAACGCA (SEQ ID Nr: 3);
GTGAGTCATCGAAACTTTGAACGCA (SEQ ID Nr: 4);
GTGAATCATCGAATCTTTGAACGCA (SEQ ID Nr: 5);
GTGAGTCATCGAATCTTTGAACGCA (SEQ ID Nr: 6);
GTGAATCATCGAAATTTTGAACGCA (SEQ ID Nr: 7);
GTGAGTCATCGAAATTTTGAACGCA (SEQ ID Nr: 8);
GTGAATCATCGAATTTTTGAACGCA (SEQ ID Nr: 9);
GTGAGTCATCGAATTTTTGAACGCA (SEQ ID Nr: 10);
GTGAATCATCGAAACTTTGAACGCA (SEQ ID Nr: 11);
GTGAGTCATCGAAACTTTGAACGCA (SEQ ID Nr: 12);
GTGAATCATCGAATCTTTGAACGCA (SEQ ID Nr: 13);
GTGAGTCATCGAATCTTTGAACGCA (SEQ ID Nr: 14);
GTGAATCATCGAAATTTTGAACGCA (SEQ ID Nr: 15);
GTGAGTCATCGAAATTTTGAACGCA (SEQ ID Nr: 16);
GTGAATCATCGAATTTTTGAACGCA (SEQ ID Nr: 17);
GTGAGTCATCGAATTTTTGAACGCA (SEQ ID Nr: 18);
oder ihre komplementären Sequenzen.

## Revendications

1. Utilisation d'au moins un acide nucléique comprenant ou constitué par :
(i) où :
R représente A ou G
Y représente C ou T
M représente A ou C
W représente A ou T
H représente A ou C ou T
(ii) une partie de SEQ ID NO : 1, à condition que ledit acide nucléique se lie dans des conditions rigoureuses à un acide nucléique comprenant ou constitué par la séquence complémentaire de SEQ ID NO : 1, ou
(iii) les séquences complémentaires de (i) et (ii) ;
en tant qu'amorce pour la détection par polymorphisme de longueur des fragments d'amplification (AFLP) ou polymorphisme de longueur des fragments de restriction terminaux (T-RFLP) d'acides nucléiques d'un ou plusieurs champignons dans un gazon ou un échantillon de sol, ledit acide nucléique étant utilisé en association avec au moins une autre amorce qui cible la région ADNr 18S /ITS1 ou la région ITS2/ADNr 28S.

2. Utilisation selon la revendication 1, d'au moins un acide nucléique comprenant ou constitué par :
(i) GTGARTCATCGAAWYTTTGAACGCA (SEQ ID NO : 2), où
R représente A ou G
Y représente C ou T
W représente A ou T
(ii) une partie de SEQ ID NO : 2, à condition que ledit acide nucléique se lie dans des conditions rigoureuses à un acide nucléique comprenant ou constitué par la séquence complémentaire de SEQ ID NO : 1, ou
(iii) les séquences complémentaires de (i) et (ii).

3. Utilisation selon la revendication 1 ou 2, dans laquelle le champignon est choisi dans le groupe constitué par *Ascochyta phleina, Curvularia affinis, Glomerella graminicola, Thanatephorus cucumeris, Pythium ultimum, Gaeumannomyces graminis, Marasmius oreades, Corticium fuciforme, Phytophthora nicotianae. Fusarium culmorum, Bipolaris sorokiniana, Microdochium nivale, Rhizoctonia cerealis, Pythium graminicola, Rhynchosporium secalis, Sclerotinia homoeocarpa, Typhula incarnate, Ustilago striiformis, Septoria macropoda.*

4. Utilisation selon l'une quelconque des revendications 1 à 3, dans laquelle l'échantillon est un échantillon de racine de gazon.

5. Utilisation selon l'une quelconque des revendications 1 à 4, dans laquelle le gazon est choisi dans le groupe constitué par les familles de *Festaceae, Aveneae, Triticeae, Chlorideae, Zoysieae, Paniceae* et *Andropogoneae.*

6. Utilisation selon l'une quelconque des revendications précédentes, dans laquelle l'autre amorce est choisie dans la liste constituée par SEQ ID NO : 39 à 42.

7. Procédé de traitement d'un gazon malade, qui comprend les étapes consistant à :
a) détecter par polymorphisme de longueur des fragments d'amplification (AFLP) ou polymorphisme de longueur des fragments de restriction terminaux (T-RFLP) l'absence ou la présence d'acides nucléiques provenant d'au moins un champignon pathogène dans un échantillon de terre dans laquelle le gazon malade pousse, ou dans un échantillon du gazon malade, avec au moins un acide nucléique tel que défini selon la revendication 1 ou 2 en tant qu'amorce, l'acide nucléique étant utilisé en association avec au moins une autre amorce qui cible la région ADNr 18S /ITS1 ou la région ITS2/ADNr 28S ;
b) si des acides nucléiques d'un ou de plusieurs champignons pathogènes ont été détectés dans l'étape a), sélectionner un ou plusieurs agents antifongiques qui ciblent le ou les champignons pathogènes dans lesquels les acides nucléiques ont été détectés ;
c) appliquer le ou les agents antifongiques sélectionnés de l'étape b) sur le gazon malade.

8. Procédé selon la revendication 7, dans lequel le champignon est choisi dans le groupe constitué par *Ascochyta phleina, Curvularia affinis, Glomerella graminicola, Thanatephorus cucumeris, Pythium ultimum, Gaeumannomyces graminis, Marasmius oreades, Corticium fuciforme, Phytophthora nicotianae. Fusarium culmorum, Bipolaris sorokiniana, Microdochium nivale, Rhizoctonia cerealis, Pythium graminicola, Rhynchosporium secalis, Sclerotinia homoeocarpa, Typhula incarnate, Ustilago striiformis, Septoria macropoda.*

9. Procédé selon la revendication 7 ou 8, dans lequel le gazon est choisi dans le groupe constitué par les familles de *Festaceae, Aveneae, Triticeae, Chlorideae, Zoysieae, Paniceae* et *Andropogoneae.*

10. Procédé selon l'une quelconque des revendications 7 à 9, dans lequel l'autre amorce est choisie dans la liste constituée par SEQ ID NO : 39 à 42.

11. Kit de détection de champignons, comprenant chacun des acides nucléiques représentés par :
(i) où :
R représente A ou G
Y représente C ou T
M représente A ou C
W représente A ou T
H représente A ou C ou T
(ii) les séquences complémentaires de (i).

12. Kit selon la revendication 11, comprenant les acides nucléiques représentés par les séquences suivantes :
GTGAATCATCGAAACTTTGAACGCA (SEQ ID NO : 3) ;
GTGAGTCATCGAAACTTTGAACGCA (SEQ ID NO : 4) ;
GTGAATCATCGAATCTTTGAACGCA (SEQ ID NO : 5) ;
GTGAGTCATCGAATCTTTGAACGCA (SEQ ID NO : 6) ;
GTGAATCATCGAAATTTTGAACGCA (SEQ ID NO : 7) ;
GTGAGTCATCGAAATTTTGAACGCA (SEQ ID NO : 8) ;
GTGAATCATCGAATTTTTGAACGCA (SEQ ID NO : 9) ;
GTGAGTCATCGAATTTTTGAACGCA (SEQ ID NO : 10) ;
GTGAATCATCGAAACTTTGAACGCA (SEQ ID NO : 11);
GTGAGTCATCGAAACTTTGAACGCA (SEQ ID NO : 12) ;
GTGAATCATCGAATCTTTGAACGCA (SEQ ID NO : 13) ;
GTGAGTCATCGAATCTTTGAACGCA (SEQ ID NO : 14) ;
GTGAATCATCGAAATTTTGAACGCA (SEQ ID NO : 15) ;
GTGAGTCATCGAAATTTTGAACGCA (SEQ ID NO : 16) ;
GTGAATCATCGAATTTTTGAACGCA (SEQ ID NO : 17) ;
GTGAGTCATCGAATTTTTGAACGCA (SEQ ID NO : 18) ;
ou leurs séquences complémentaires.


### SEQUENCE LISTING

<110> Omya Development AG
<120> NUCLEIC ACIDS AND METHODS FOR DETECTING TURFGRASS PATHOGENIC FUNGI
<130> BET 09P0535
<150> EP 08104225.1 2008-06-02
<150> US 61/059,862 2008-06-09
<160> 42
<170> PatentIn version 3.4
<210> 1
   <211> 86
   <212> DNA
   <213> Artificial sequence
<220>
   <223> Fungal consensus sequence
<400> 1
   catcgatgaa gaacgcwgcr aahtgcgata mgtartgyga attgcagrat tcagtgartc 60
   atcgaawytt tgaacgcaym ttgcrc 86
<210> 2
   <211> 25
   <212> DNA
   <213> Artificial sequence
<220>
   <223> Degenerated PCR primer for fungal detection
<400> 2
   gtgartcatc gaawytttga acgca 25
<210> 3
   <211> 25
   <212> DNA
   <213> Artificial sequence
<220>
   <223> PCR primer for fungal detection
<400> 3
   gtgaatcatc gaaactttga acgca 25
<210> 4
   <211> 25
   <212> DNA
   <213> Artificial sequence
<220>
   <223> PCR primer for fungal detection
<400> 4
   gtgagtcatc gaaactttga acgca 25
<210> 5
   <211> 25
   <212> DNA
   <213> Artificial sequence
<220>
   <223> PCR primer for fungal detection
<400> 5
   gtgaatcatc gaatctttga acgca 25
<210> 6
   <211> 25
   <212> DNA
   <213> Artificial sequence
<220>
   <223> PCR primer for fungal detection
<400> 6
   gtgagtcatc gaatctttga acgca 25
<210> 7
   <211> 25
   <212> DNA
   <213> Artificial sequence
<220>
   <223> PCR primer for fungal detection
<400> 7
   gtgaatcatc gaaattttga acgca 25
<210> 8
   <211> 25
   <212> DNA
   <213> Artificial sequence
<220>
   <223> PCR primer for fungal detection
<400> 8
   gtgagtcatc gaaattttga acgca 25
<210> 9
   <211> 25
   <212> DNA
   <213> Artificial sequence
<220>
   <223> PCR primer for fungal detection
<400> 9
   gtgaatcatc gaatttttga acgca 25
<210> 10
   <211> 25
   <212> DNA
   <213> Artificial sequence
<220>
   <223> PCR primer for fungal detection
<400> 10
   gtgagtcatc gaatttttga acgca 25
<210> 11
   <211> 25
   <212> DNA
   <213> Artificial sequence
<220>
   <223> PCR primer for fungal detection
<400> 11
   gtgaatcatc gaaactttga acgca 25
<210> 12
   <211> 25
   <212> DNA
   <213> Artificial sequence
<220>
   <223> PCR primer for fungal detection
<400> 12
   gtgagtcatc gaaactttga acgca 25
<210> 13
   <211> 25
   <212> DNA
   <213> Artificial sequence
<220>
   <223> PCR primer for fungal detection
<400> 13
   gtgaatcatc gaatctttga acgca 25
<210> 14
   <211> 25
   <212> DNA
   <213> Artificial sequence
<220>
   <223> PCR primer for fungal detection
<400> 14
   gtgagtcatc gaatctttga acgca 25
<210> 15
   <211> 25
   <212> DNA
   <213> Artificial sequence
<220>
   <223> PCR primer for fungal detection
<400> 15
   gtgaatcatc gaaattttga acgca 25
<210> 16
   <211> 25
   <212> DNA
   <213> Artificial sequence
<220>
   <223> PCR primer for fungal detection
<400> 16
   gtgagtcatc gaaattttga acgca 25
<210> 17
   <211> 25
   <212> DNA
   <213> Artificial sequence
<220>
   <223> PCR primer for fungal detection
<400> 17
   gtgaatcatc gaatttttga acgca 25
<210> 18
   <211> 25
   <212> DNA
   <213> Artificial sequence
<220>
   <223> PCR primer for fungal detection
<400> 18
   gtgagtcatc gaatttttga acgca 25
<210> 19
   <211> 589
   <212> DNA
   <213> Ascochyta phleina
<400> 19
   tccgtaggtg aacctgcgga aggatcatta cactcagtag tttactactg taaaggaggc     60
   cgtcagtctg tatagcttca tcgctgacga gcagctggtc tcttttatcc acccttgtct    120
   tttgcgtacc catgtttcct cggcaggctt gcctgccgac tggacaaatt tataaccttt    180
   ttaattttca atcagcgtct gaaaaactta ataattacaa ctttcaacaa cggatctctt    240
   ggttctggca tcgatgaaga acgcagcgaa atgcgataag tagtgtgaat tgcagaattc    300
   agtgaatcat cgaatctttg aacgcacatt gcgccccttg gtattccatg gggcatgcct    360
   gttcgagcgt catttgtacc ctcaagcttt gcttggtgtt gggtgtttgt cctctccctg    420
   gtgtttggac tcgccttaaa acaattggca gccagtgttt tggtattgaa gcgcagcaca    480
   agtcgcgatt ctaacctagt aacacttgcg tccacaagcc ttttttcact tttgacctcg    540
   gatcaggtag ggatacccgc tgaacttaag catatcaata agcggagga                589
<210> 20
   <211> 584
   <212> DNA
   <213> Curvularia affinis
<400> 20
   tccgtaggtg aacctgcgga gggatcatta cacaataaac atatgaaggc tgcaccgcca     60
   gttggcggca aggctggagt attttattac ccttgtcttt tgcgcacttg ttgtttcctg    120
   ggcgggttcg cccgccacca ggaccacatg ataaaccttt tttatgcagt tgcaatcagc    180
   gtcagtacaa caaatgtaaa tcatttacaa ctttcaacaa cggatctctt ggttctggca    240
   tcgatgaaga acgcagcgaa atgcgatacg tagtgtgaat tgcagaattc agtgaatcat    300
   cgaatctttg aacgcacatt gcgccctttg gtattccaaa gggcatgcct gttcgagcgt    360
   catttgtacc ctcaagcttt gcttggtgtt gggcgttttg tctttggttg ccaaagactc    420
   gccttaaaac gattggcagc cggcctactg gtttcgcagc gcagcacatt tttgcgcttg    480
   caatcagcaa aagaggacgg cactccatca agactcttta tcacttttga cctcggatca    540
   ggtagggata cccgctgaac ttaagcatat caataagcgg agga                      584
<210> 21
   <211> 587
   <212> DNA
   <213> Glomerella graminicola
<400> 21
   tccgtaggtg aacctgcgga gggatcatta ctgagttacc gctctataac cctttgtgaa     60
   catacctaac cgttgcttcg gcgggttagg gggtcccctc tccgggggac gccctcccgg    120
   ccgggcccca ctgcggggct cggcgcccgc cggaggataa ccaaactctg atttaacgac    180
   gtctcttctg agtggcacaa gcaaataatt aaaactttta acaacggatc tcttggttct    240
   ggcatcgatg aagaacgcag cgaaatgcga taagtaatgt gaattgcaga attcagtgaa    300
   tcatcgaatc tttgaacgca cattgcgccc gccagcattc tggcgggcat gcctgttcga    360
   gcgtcatttc aaccctcaag ctccgcttgg tgttggggcc ctacggcgta cgtcgtaggc    420
   ccttaaaggt agtggcggac cctcccggag cctcctttgc gtagtaacta acgtctcgca    480
   tcgggatccg gagggactct tgccgtaaaa cccccaactt tttaactggt tgacctcgga    540
   tcaggtagga atacccgctg aacttaagca tatcaataag cggagga                   587
<210> 22
   <211> 710
   <212> DNA
   <213> Thanatephorus cucumeris
<400> 22
   tccgtaggtg aacctgcgga aggatcatta ttgaatttta ttaatgagga gttgagttgt     60
   tgctggcctt ttctacctta atttggcagg agggggcatg tgcacacctt ctcttttatc    120
   catcacaccc cctgtgcact tgtgagacag caatagttgg tggatttaat tccatccatt    180
   tgctgtctac ttaatttaca cacactctac ttaatttaaa ctgaatgtaa ttgatgtaac    240
   gcatctaata ctaagtttca acaacggatc tcttggctct cgcatcgatg aagaacgcag    300
   cgaaatgcga taagtaatgt gaattgcaga attcagtgaa tcatcgaatc tttgaacgca    360
   ccttgcgctc cttggtattc cttggagcat gcctgtttga gtatcatgaa atcttcaaag    420
   taaacctttt gttaattcaa ttggtctttt ttactttggt tttggaggat cttattgcag    480
   cttcacacct gctcctcttt gtgcattagc tggatctcag tgttatgctt ggttccactc    540
   ggcgtgataa gttatctatc gctgaggaca cccgtaaaaa aggtggccaa ggtaaatgca    600
   gatgaaccgc ttctaatagt ccattgactt ggacaatatt ctattttatg atctgatctc    660
   aaatcaggta ggactacccg ctgaacttaa gcatatcaat aagcggagga                710
<210> 23
   <211> 914
   <212> DNA
   <213> Pythium ultimum
<400> 23
   tccgtaggtg aacctgcgga aggatcatta ccacacttta aaaaactgtc cacgtgaact     60
   gtaagcaagt ctagcgctgt gactgagctg gtgttttcat ttttggacac tggaacggga    120
   gtcagcagga cgaaggttgg tctgttgtaa tgcaagttat gatggactag ctgatgaact    180
   tttgttttta aacccttacc taaatactga tttatactgt ggggacgaaa gtccttgctt    240
   ttactagata acaactttca gcagtggatg tctaggctcg cacatcgatg aagaacgctg    300
   cgaactgcga tacgtaatgc gaattgcaga attcagtgag tcatcgaaat tttgaacgca    360
   tattgcactt tcgggttatg cctggaagta tgtctgtatc agtgtccgta aatcaaactt     420
   gcctttcttt ttccgtgtag tcagggatgg aatgtgcaga tgtgaagtgt ctcgcatggt     480
   tgcgttcgtt ttttcgatcg agaatctgtc gagtcctttt aaatggacac ggtcttttct     540
   atggtttcta tgaagtgtaa tggttggaag gcagtgattt tcggattgct ggcggctttt     600
   ggcgacttcg gtatgaacgt atggagacta gctcaattcg tggtatgtta ggcttcggct     660
   cgacaatgtt gcgtaattgt gtgtggtctt tgtttgtgcc ttgaggtgta ctagaggttg     720
   tcggtttgaa ccgtaagtga ttgtttagta gagcattttc acgatgtatg gagacgctgc     780
   atttagttgc gtagagagat tgatttggga aattttgtat cattgtcaat tgcaagattg     840
   tgtatggtat ctcaattgga cctggtatca gacaagacta cccgctgaac ttaagcatat     900
   caataagcgg agga                                                       914
<210> 24
   <211> 558
   <212> DNA
   <213> Gaeumannomyces graminis
<400> 24
   tccgtaggtg aacctgcgga gggatcatta cagagttgaa aaactccaac ccctgtgaac      60
   atacctttac tgttgcttcg gcggacgatg gccccccccg ggggccggac gccgccggag     120
   gttacaaacc ctgaatttta gtgtatctct gagtataaaa ccaaataatt aaaactttca     180
   acaacggatc tcttggttct ggcatcgatg aagaacgcag cgaaatgcga taagtaatgt     240
   gaattgcaga attcagtgaa tcatcgaatc tttgaacgca cattgcgccc gccggtattc     300
   cggcgggcat gcctgtccga gcgtcatttc accactcaag cccagcttgg tgttggggca     360
   cccggccgcc cggcggtcgg ggcccccaag aacatcggcg gtctcgccag gaccctgaac     420
   gcagtaactc gcggtaaaac gcgcttcgtt cggaggcttc ccggcgggct ccagccgcta     480
   aaccccctaa acttcttagg ttgacctcgg atcaggtagg aatacccgct gaacttaagc     540
   atatcaataa gcggagga                                                   558
<210> 25
   <211> 672
   <212> DNA
   <213> Marasmius oreades
<400> 25
   tccgtaggtg aacctgcgga aggatcatta ttgaaacatt gtaaaggaag gttgagctgg    60
   ctcttcacgg gcatgtgctc gcctttcttt caatcttcat ccacctgtgc actttttgta   120
   gggagctttg agaatgggac ctctcgcggg gttcctagta ttgggctctc tatgtcttca   180
   cacactcttg aatgtatgtc gttgaatgtc ttttacaggg acttaattga ccctttaaaa   240
   actatacaac tttcagcaac ggatctcttg gctctcgcat cgatgaagaa cgcagcgaaa   300
   tgcgataagt aatgtgaatt gcagaattca gtgaatcatc gaatctttga acgcaccttg   360
   cgcctcttgg tattccgaga ggcatgcctg tttgagtgtc attaaattct caacttcaaa   420
   agcttttgtt tttgaagctt ggatgtggag gctttgctgg ctcttctaga gtcggctcct   480
   ctgaaatgca ttagtggaaa ctgtttgcaa tccgcattgg tgtgataatt atctacgctt   540
   gtgtgtggct gcagctcttt acgagtttag tatccgcttc aaaccgtcct aagttactgg   600
   acaacttgaa ccttttgacc tcaaatcagg taggactacc cgctgaactt aagcatatca   660
   ataagcggag ga                                                       672
<210> 26
   <211> 716
   <212> DNA
   <213> Corticium fuciforme
<400> 26
   tccgtaggtg aacctgcgga aggatcatta acgagtttta aaagagttgt agctggccct    60
   ctggggtatg tgcacgctct actcatccac atacacctgt gcacatagat agtctttttt   120
   ttgagaaggg ggagaaaaag tagccctttg ggtgaaaagt ttcccccctc ttcagaaagc  180
   tattcttttt atacacatac actttagtta agaatgtata tattgctata aaacgcatta   240
   aatataactt tcaacaacgg atctcttggc tctcgcatcg atgaagaacg cagcgaaatg   300
   cgataagtaa tgtgaattgc agaattcagt gaatcatcga atctttgaac gcaccttgcg   360
   ctccttggta ttccgaggag catgcctgtt tgagtgtcat gaatatctca actctcaaag   420
   ttctgtaatg gatcattgag agcttggact ttggaggctt gctggtcatc atgtatcagc   480
   tcctcttaaa tgaattagct ctggattacg atgtagttta tctacggtgt gatacatgtc   540
   tacgctatta gataactacg aacgttacaa ttcctccatt tctggggttt gcgtgactag   600
   taaatacaaa gcttctaatt gtctttcggg acaaaacctg atttcattat cttgacatct   660
   gacctcaaat caggtaggac tacccgctga acttaagcat atcaataagc ggagga       716
<210> 27
   <211> 892
   <212> DNA
   <213> Phytophthora nicotianae
<400> 27
   tccgtaggtg aacctgcgga aggatcatta ccacacctaa aaaactttcc acgtgaaccg     60
   tttcaaccca atagttgggg gtcttatttg gcggcggctg ctggcttaat tgttggcggc    120
   tgctgctgag tgagccctat caaaaaaaag gcgaacgttt gggcttcggc ctgatttagt    180
   agtctttttt tcttttaaac ccattcctta atactgaata tactgtgggg acgaaagtct    240
   ctgcttttaa ctagatagca actttcagca gtggatgtct aggctcgcac atcgatgaag    300
   aacgctgcga actgcgatac gtaatgcgaa ttgcaggatt cagtgagtca tcgaaatttt    360
   gaacgcatat tgcacttccg ggttagtcct ggaagtatgc ctgtatcagt gtccgtacat    420
   taaacttgac tttcttcctt ccgtgtagtc ggtggaggag atgtcagatg tgaagtgtct    480
   tgcgattggt cttcggaccg gctgcgagtc cttttaaatg tactaaactg aacttctctt    540
   tgctcgaaaa gtggtggcgt tgctggttgt gaaggctgct attgtggcaa attggcgact    600
   ggtttgtctg ctgcggcgtt aatgggagag tgttcgattc gtggtatggt tggcttcggc    660
   tgaacaatgc acttattgga cgtttttcct gctgtggcgt gatggactgg tgaaccatag    720
   ctcggtggct tggcttttga attggctttg ctgttgcgaa gtagggtggc agcttcggtt    780
   gtcgagggtc gatccatttg ggaacttaat gtgtacttcg gtatgcatct caattggacc    840
   tgatatcagg caagattacc cgctgaactt aagcatatca ataagcggag ga            892
<210> 28
   <211> 545
   <212> DNA
   <213> Fusarium culmorum
<400> 28
   tccgtaggtg aacctgcgga gggatcatta ccgagtttac aactcccaaa cccctgtgaa      60
   cataccttat gttgcctcgg cggatcagcc cgcgccccgt aaaaagggac ggcccgccgc     120
   aggaacccta aactctgttt ttagtggaac ttctgagtat aaaaaacaaa taaatcaaaa     180
   cttccaacaa cggatctctt ggttctggca tcgatgaaga acgcagcaaa atgcgataag     240
   taatgtgaat tgcagaattc agtgaatcat cgaatctttg aacgcacatt gcgcccgcca     300
   gtattctggc gggcatgcct gttcgagcgt catttcaacc ctcaagccca gcttggtgtt     360
   gggagctgca gtcctgctgc actccccaaa tacattggcg gtcacgtcga gcttccatag     420
   cgtagtaatt tacatatcgt tactggtaat cgtcgcggcc acgccgttaa accccaactt     480
   ctgaatgttg acctcggatc aggtaggaat acccgctgaa cttaagcata tcaataagcg     540
   gagga                                                                 545
<210> 29
   <211> 586
   <212> DNA
   <213> Bipolaris sorokiniana
<400> 29
   tccgtaggtg aacctgcgga gggatcatta cacaacaaaa tatgaaggcc tggcttcgcg      60
   gccggctgaa atattttttt cacccatgtc ttttgcgcac ttgttgtttc ctgggcgggt     120
   tcgcccgcca ccaggaccaa accataaacc ttttttttat gcagttgcaa tcagcgtcag     180
   taaaaacaat gtaattatta caactttcaa caacggatct cttggtcctg gcatcgatga     240
   agaacgcagc gaaatgcgat acgtagtgtg aattgcagaa ttcagtgaat catcgaatct     300
   ttgaacgcac attgcgccct ttggtattcc aaagggcatg cctgttcgag cgtcatttgt     360
   accttcaagc tttgcttggt gttgggcgtt ttttgtctcc ctctttctgg gagactcgcc     420
   ttaaaacgat tggcagccgg cctactggtt tcggagcgca gcacatattt tgcgctttgt     480
   atcaggagaa aaggacggta atccatcaag actctacatt tttaactttt gacctcggat     540
   caggtaggga tacccgctga acttaagcat atcaataagc ggagga                    586
<210> 30
   <211> 556
   <212> DNA
   <213> Microdochium nivale
<400> 30
   tccgtaggtg aacctgcgga gggatcatta ctgagttttt aactctccaa accatgtgaa     60
   cttaccactg ttgcctcggt ggatggtgct gtctctcggg acggtgccac cgccggtgga    120
   ctacctaaac tctgttaatt tttgtcaatc tgaatcaaac taagaaataa gttaaaactt    180
   tcaacaacgg atctcttggt tctggcatcg atgaagaacg cagcgaaatg cgataagtaa    240
   tgtgaattgc agaattcagt gaatcatcga atctttgaac gcacattgcg cccattagta    300
   ttctagtggg catgcctgtt cgagcgtcat ttcaaccctt aagcctagct tagtgttggg    360
   agactgccta atacgcagct cctcaaaacc agtggcggag tcggttcgtg ctctgagcgt    420
   agtaattttt tatctcgctt ctgcaagccg gactggcaac agccataaac cgcacccttc    480
   gggggcactt tttaatggtt gacctcggat caggtaggaa tacccgctga acttaagcat    540
   atcaataagc ggagga                                                    556
<210> 31
   <211> 684
   <212> DNA
   <213> Rhizoctonia cerealis
<400> 31
   tccgtaggtg aacctgcgga aggatcatta gtgaatgaat gtagagtcgg ttgtagctgg     60
   gtcttttaat cgaggccatg tgcacgcctt ctctttcatc cacacacacc tgtgcacctg    120
   tttagacggt cgaaggaaaa agtctttctc gcgagagaga ggctggctcc ttttccgtcc    180
   aatacataaa atcttatata tttaatcaga atgtaatcga tgtaaacgca tctataaact    240
   aagtttcaac aacggatctc ttggctctcg catcgatgaa gaacgcagcg aaatgcgata    300
   agtaatgtga attgcagaat tcagtgaatc atcgaatctt tgaacgcacc ttgcgctcct    360
   tggtattcct cggagcacgc ctgtttgagt atcatgaaat tctcaaagca agtcttttgt    420
   taattcaact ggcttttgtt ttggatttgg aggttttgca gattcacgtc tgctcctctt    480
   aaatgcatta gctggatctc tataaaaccg gttccactcg gcgtgataag tatcactcgc    540
   tgaggacact cttgaaaaag ggtggccgga ttcatggatg aaccgcttct aacggtctat    600
   tagattagac aaacacactt tatgatctga tctcaaatca ggtgggacta cccgctgaac    660
   ttaagcatat caataagcgg agga                                           684
<210> 32
   <211> 872
   <212> DNA
   <213> Pythium graminicola
<400> 32
   tccgtaggtg aacctgcgga aggatcatta ccacaccaaa aaactttcca cgtgaaccgt      60
   tataattatg ttctgtgctt tccttcggga aggctgaacg aaggttgatc gtatgtatta     120
   atttatgtgt ggtcttccga tgtcttttaa acccattact taatactgat ctatactccg     180
   agaacgaaag tttttggttt taatccataa caactttcag cagtggatgt ctaggctcgc     240
   acatcgatga agaacgctgc gaactgcgat acgtaatgcg aattgcagaa ttcagtgagt     300
   catcgaaatt ttgaacgcac attgcacttt cgggatattc ctggaagtat gcttgtatca     360
   gtgtccgtac atcaaacttg cctttctttt tttgtgtagt caaggagaga aatggccgat     420
   tgtgaggtgt ctcgttgact cccttttcgg aggagaagac gcgagtccct ttaaatgtac     480
   gttcgctctt tcttgtgtct gaggtgaagt gtgactttcg aacgcattga tctgtttgga     540
   tcgttttgcg cgagtgggcg acttcggtta ggacgttaaa ggaagcaacc attattggcg     600
   gtatgttaga cttcggtccg actttgcagc tgagagtgtg tagttttctg ttctttcctt     660
   gaggtgtacc tgtttgtgtg aggcaatggt ctgagcaaat ggttattgtg tagtagaatt     720
   ttgctgctct tggacgccct attcgtaggg taaagtagac aacaccattt tgggactagt     780
   ctatgtatta atttatgtgg gcgatttttc aatttggacc tgatatcaag taagactacc     840
   cgctgaactt aagcatatca ataagcggag ga                                   872
<210> 33
   <211> 627
   <212> DNA
   <213> Rhynchosporium secalis
<400> 33
   tccgtaggtg aacctgcgga aggatcatta atagagcaaa gaacagtcag cgccctagga      60
   gaaatcccgg gggctaccct acttcggtgg ggtttagaga cgtcaggccg ctcgaagaag 120
   cctggttcag acctccaccc ttgaataaac tacctttgtt gctttggcag gccgcccagc 180
   gccagcggct tcggctgctg agtgcctgcc agaggaccac aactcttgtt tttagtgatg 240
   tctgagtact atataatagt taaaactttc aacaacggat ctcttggttc tggcatcgat 300
   gaagaacgca gcgaaatgcg ataagtaatg tgaattgcag aattcagtga atcatcgaat 360
   ctttgaacgc acattgcgcc ctctggtatt ccggggggca tgcctgttcg agcgtcatta 420
   taaccactca agctctcgct tggtattgga gttcgcgtcc tcgcggcccc taaaatcagt 480
   ggcggtgcct gtcggctcta cgcgtagtaa tactcctcgc gattgagtcc ggctggtcta 540
   cttgccaata acccccaaat ttttacaggt tgacctcgga tcaggtaggg atacccgctg 600
   aacttaagca tatcaataag cggagga 627
<210> 34
   <211> 586
   <212> DNA
   <213> Sclerotinia homoeocarpa
<400> 34
   tccgtaggtg aacctgcgga aggatcatta ccgagttcac gccctcacgg gtagacctcc 60
   aacccttgtg tatctctacc atgttgcttt ggcaggctgc tcgacccttc cggggacagc 120
   ctcagcgccc tccggggccg gagagtcgcc tgccggagga aaatcacaac tctgaattgt 180
   cagtgtcgtc tgagtgacta tctaatagtt aaaactttca acaacggatc tcttggttct 240
   ggcatcgatg aagaacgcag cgaaatgcga taagtaatgt gaattgcaga attcagtgaa 300
   tcatcgaatc tttgaacgca cattgcgccc cttggtattc cggggggcat gcctgttcga 360
   gcgtcatttc aaccctcaag ctctctgctt ggtattgggc ctccgccggt cacacggcgg 420
   gccttaaagt cagtggcggc gccgctgggt cctgaacgta gtaacacata cctctcgtta 480
   cagggtcccc gcgcgctccc gccgtaaaac ccccctcatt ttctctggtt gacctcggat 540
   caggtaggga tacccgctga acttaagcat atcaataagc ggagga 586
<210> 35
   <211> 825
   <212> DNA
   <213> Typhula incarnate
<400> 35
   tccgtaggtg aacctgcgga aggatcatta tcgaatttaa ggctttggtt gagctggcgc     60
   ttcggtgcat gtgcttgcct tgtgctgtcc attcttcaac acctgtgcac actttgtagt    120
   tgactctttt gtttatctgt tcatctctct cttgactccg gtctctgtga aggggtgcgt    180
   ggcttttcga aagcaaggtc ctctatgtta ttattataca ccctttacaa aaaacaagtc    240
   catagaacgt ccaatgtagg cgcagcgtaa aacactgttt gctgaaatta taaaacttat    300
   acaactttta acaacggatc tcttggctct cgcatcgatg aagaacgcag cgaaatgcga    360
   taagtaatgt gaattgcaga attcagtgaa tcatcgaatc tttgaacgca ccttgcgctc    420
   cctggtattc cggggagcat gcctgtttga gtgtcattaa attctcaacc acactatgtt    480
   tttattaacg tagttcttgt ggcttggatc ttggagtttg tgccggtaaa cctttagtta    540
   ggttgttggc tcctctttaa atgcattagc tggaacctct ttgtggtgcc agactatggt    600
   gtgataatta tctacgctgt ggtttgttgc gctgcgaatt taactatggg gttctgcttc    660
   taatcgtccc tttcaaagga cagtatagag tgtggtgggg tgggggttgc tttcaagggg    720
   ttcgcctctt gtgttacaat cttgcccttt acctcttaat tgacattttg acctcaaatc    780
   aggtaggact acccgctgaa cttaagcata tcaataagcg gagga                    825
<210> 36
   <211> 780
   <212> DNA
   <213> Ustilago striiformis
<400> 36
   tccgtaggtg aacctgcgga tggatcattt cgatgaaaac ctttttttct tgaggtgtgg 60
   ctcgcacctg tctaactaaa cttgagctac cttttttcaa cacggttgca tcggttggcc 120
   tgtcaaacag tgcggcggcg tgaattttca cgtctgcttt ggctgggcga cggaccgaca 180
   cttaatcaac acttttgata atctaggatt tgaatgataa aagttcattt ttacaatgaa 240
   atcgactggt aatgcggtcg tctaattttt aaaaacaact tttggcaacg gatctcttgg 300
   ttctcccatc gatgaagaac gcagcgaatt gcgataagta atgtgaattg cagaagtgaa 360
   tcatcgaatc tttgaacgca ccttgcgctc ccggcagatc taatctgggg agcatgcctg 420
   tttgagggcc gcgaattgtt tcgaacgaca actttttctc tctttttttt gaagagttgg 480
   cggatcggta ttgagggttt tgccattcac cgtggctccc tcgaaatgca ttagcgcatc 540
   catttgatag gcaagacgga cgaaagctcg attttcgctc tctcttccct gccgggtttc 600
   gataatatca ggacttcgga gaggttgaga tgggtaagag ctggacgcaa cggcttgctg 660
   tttggagtgc ttctgaaacc cgcccatatc gagctttgcc tcggaaggga ctttaataat 720
   tcatcggcct cagattggta ggactacccg ctgaacttaa gcatatcaat aagcggagga 780
<210> 37
   <211> 540
   <212> DNA
   <213> Septoria macropoda
<400> 37
   tccgtaggtg aacctgcgga gggatcatta ccgagtgggg gcctccgggt ccgatctcca 60
   accctttgtg aacacatccc gttgcttcgg gggcgcccgg gccgggcgcc cccggaggac 120
   catccaacac tgcatctttg cgtcggagtt tacgagtaaa tcgaaacaaa actttcaaca 180
   acggatctct tggttctggc atcgatgaag aacgcagcga aatgcgataa gtaatgtgaa 240
   ttgcagaatt cagtgaatca tcgaatcttt gaacgcacat tgcgccccct ggtattccgg 300
   ggggcatgcc cgttcgagcg tcattacacc actccagcct cgctgggtat tgggcgtccg 360
   cgggggagca atcccccgcg cgcctcaaag tctccggctg agcggtcccg tctcccagcg 420
   ttgtggcatc acgtctcgcc gcggagtctc gggccctcac ggccgttaaa tcacacctca 480
   ggttgacctc ggatcgggta gggatacccg ctgaacttaa gcatatcaat aagcggagga 540
<210> 38
   <211> 25
   <212> DNA
   <213> Artificial sequence
<220>
   <223> Degenerated PCR primer for fungal detection
<400> 38
   tgcgttcaaa rwttcgatga ytcac 25
<210> 39
   <211> 19
   <212> DNA
   <213> Artificial sequence
<220>
   <223> ITS1-F primer
<400> 39
   tccgtaggtg aacctgcgg 19
<210> 40
   <211> 20
   <212> DNA
   <213> Artificial sequence
<220>
   <223> ITS4 primer
<400> 40
   tcctccgctt attgatatgc 20
<210> 41
   <211> 22
   <212> DNA
   <213> Artificial sequence
<220>
   <223> ITS5 primer
<400> 41
   ggaagtaaaa gtcgtaacaa gg 22
<210> 42
   <211> 20
   <212> DNA
   <213> Artificial sequence
<220>
   <223> SR6R primer
<400> 42
   aagwaaaagt cgtaacaagg 20